(19) Europäisches Patentamt

European Patent Office

Office européen des brevets

(11) Veröffentlichungsnummer: **0 255 655 B1**

(12) **EUROPÄISCHE PATENTSCHRIFT**

(45) Veröffentlichungstag der Patentschrift: **19.11.92**

(21) Anmeldenummer: **87110602.7**

(22) Anmeldetag: **22.07.87**

(51) Int. Cl.⁵: **A61K 7/035**, B65D 81/32

(54) Verfahren zur Herstellung von verschiedenfarbigen festen Puder-Zubereitungen.

(30) Priorität: **05.08.86 EP 86110823**

(43) Veröffentlichungstag der Anmeldung:
**10.02.88 Patentblatt 88/06**

(45) Bekanntmachung des Hinweises auf die
Patenterteilung:
**19.11.92 Patentblatt 92/47**

(84) Benannte Vertragsstaaten:
**AT BE CH DE ES FR GB GR IT LI LU NL SE**

(56) Entgegenhaltungen:
**EP-A- 0 135 060**
**DE-A- 2 349 051**

(73) Patentinhaber: **Württembergische Parfümerie-Fabrik GmbH**
**Schillerstrasse 21-27**
**W-7332 Eislingen/Fils 16(DE)**

(72) Erfinder: **Scheller, Hans Ulrich, Dr.**
**Vogelgartenstrasse 45**
**W-7332 Eislingen/Fils(DE)**
Erfinder: **Scheller, Karl Alexander**
**Kelterstrasse 38**
**W-7332 Eislingen/Fils(DE)**

(74) Vertreter: **Werner, Hans-Karsten, Dr. et al**
**Deichmannhaus am Hauptbahnhof**
**W-5000 Köln 1(DE)**

**Beschreibung**

Gegenstand der vorliegenden Erfindung ist ein Verfahren zur Herstellung von Behältnissen mit zwei oder mehreren voneinander getrennten verschiedenfarbigen Teilbereichen fester, gegossener Puder-Zubereitungen. Insbesondere handelt es sich um voneinander getrennte verschiedenfarbige Teilbereiche fester, gegossener Puder-Zubereitungen gemäß EP-A-135 060.

Behältnisse mit zwei oder mehreren voneinander getrennten verschiedenfarbigen Teilbereichen fester, gegossener Puder-Zubereitungen konnten bisher nur hergestellt werden, wenn mehrere entsprechend aneinanderpassende Behältnisse vorhanden waren oder in die Behältnisse vorher Trennstege eingebaut oder eingespritzt worden waren. Dies erfordert zumindest einen entsprechend hohen Aufwand für Spritzwerkzeuge bzw. die Montage von mehreren Aufnahmeformen (Pfannen) in die Behältnisse.

Die Erfindung hat sich die Aufgabe gestellt, ein Verfahren zu entwickeln, durch welches zwei oder mehrere voneinander getrennte verschiedenfarbige Teilbereiche fester Puder-Zubereitungen in Behältnisse gegossen werden können, ohne daß die Farben zusammenfließen und sich vermischen. Weiterhin bestand die Aufgabe, eine qualitative Veränderung der festen, gegossenen Puder-Zubereitungen zu vermeiden. Schließlich sollte das Verfahren möglichst einfach aber flexibel anwendbar sein, um nicht nur gerade sondern auch gekrümmte und geschwungene Trennlinien zwischen den verschiedenfarbigen Teilbereichen der Puder-Zubereitungen zu ermöglichen.

Diese Aufgabe konnte überraschend einfach dadurch gelöst werden, daß man spritzfähige pastöse Massen entwickelt hat, die eine ähnliche Zusammensetzung aufweisen wie die fertigen festen gegossenen Puder-Zubereitungen und diese Massen dann als Trennstege in die Behältnisse einspritzt. Nach dem Einspritzen der pastösen Stege können in die einzelnen Teilbereiche der durch Stege unterteilten Behältnisse verschiedenfarbige Puder-Zubereitungen gegossen werden, woraufhin in üblicher Weise der Überschuß des Lösungsmittel verdampft und gewünschtenfalls die Oberfläche der so erhaltenen Produkte mit Drücken unter 10 bar bearbeitet wird.

Gegenstand der vorliegenden Erfindung ist somit ein Verfahren zur Herstellung von Behältnissen mit zwei oder mehreren voneinander getrennten, verschiedenfarbigen Teilbereichen fester, gegossener Puder-Zubereitungen, dadurch gekennzeichnet, daß in die Behältnisse

I. Trennstege eingespritzt werden aus einer pastösen Masse bestehend aus

    a) 0 bis 15 Gew.-% eines nicht hygroskopischen, fähigen, organischen Feststoffs mit einer Teilchengröße von 5 bis 50 $\mu$m Durchmesser als Füllstoff,

    b) einem oder mehreren fettartigen und wachsartigen Bestandteilen,

    c) einem farbneutralen Pigment oder Perlpigment,

    d) einem oder mehreren verdampfbaren untoxischen, hydrophoben Lösungsmitteln,

    e) gegebenenfalls weiteren üblichen Zusätzen für gießbare feste Puder-Zubereitungen,

worauf

II. in die einzelnen Teilbereiche der durch Stege unterteilten Behältnisse verschiedenfarbige Puder-Zubereitungen gegossen werden,

worauf

III. der Überschuß des Lösungsmittels verdampft und gewünschtenfalls die Oberfläche der so erhaltenen Produkte mit Drücken unter 10 bar bearbeitet wird.

Da die Zusammensetzung der Zusammensetzung inzwischen handelsüblicher fester, gegossener Pulver-Zubereitungen gemäß EP-A-135 060 entsprechen soll, wird vorzugsweise Füllstoff Nylon-12-Pulver verwendet, bestehend aus überwiegend kugelförmigen, ellipsoiden Körpern. Das Schüttgewicht beträgt meist 0,20 bis 0,35. Die Dichte liegt meist zwischen 1,02 bis 1,03. Eingesetzt wird dieses Nylon-12-Pulver in Mengen zwischen 7,0 bis 15 Gew.-%, vorzugsweise ca. 8 Gew.-%. Insbesondere wenn als weitere Komponente Polysiloxan-polyalkyl-polyethercopolymere in Mengen von 10 bis 20 Gew.-% eingesetzt werden, können die Füllstoffe gemäß Merkmal a) völlig entfallen.

Als fettartige und wachsartige Bestandteile werden vorzugsweise Oleyl Erucate, Stearinsäure-mono-ethanolamid und Glyceryl Oleate verwendet. Die Mengen liegen vorzugsweise zwischen 3,0 bis 5, insbesondere ca. 4 Gew.-% Oleyl Erucat, 0,8 bis 2,0 Gew.-%, insbesondere ca. 1 Gew.-% Stearinsäure-mono-ethanolamid, 0,4 bis 2,0 Gew.-%, insbesondere ca. 0,5 Gew.-% Glycerylstearat und/oder Oleat. Weiterhin kommen Gemische aus Bienenwachs, Stearinsäure-mono-ethanolamid und mikrokristallinem Hartwachs in Frage. Sie werden meist in Mengen von je 6 bis 8 Gew.-% eingesetzt.

Die Mengen an farbneutralem Pigment bzw. Perlpigment liegen vorzugsweise zwischen 40 und 50 Gew.-%, insbesondere bei ca. 43 Gew.-%. Insbesondere wenn keine Füllstoffe gemäß Merkmal a) eingesetzt werden, kann auch die Menge an farbneutralem Pigment bzw. Perlpigment auf 8 bis 15 Gew.-% gesenkt werden.

Als verdampfbares untoxisches hydrophobes Lösungsmittel wird vorzugsweise Octamethylcyclotetrasiloxan eingesetzt und zwar in Mengen von 35,0 bis 45,0 Gew.-%. Wenn keine Füllstoffe nach Merkmal a) vorhanden sind, kann der Gehalt an Lösungsmittel auf 50 bis 60 Gew.-% erhöht werden.

Als weitere übliche Zusätze für gießbare feste Puder-Zubereitungen werden auch erfindungsgemäß ca. 0,1 Gew.-% Methylparaban, ca. 0,1 Gew.-% Propylparaban, ca. 0,1 Gew.-% Methylchloroisothiazolinone und Methylisothiazolinone sowie ca. 0,05 Gew.-% Butylhydroxytolulol eingesetzt. Wenn auf den Zusatz von Füllstoffen gemäß Merkmal a) verzichtet werden soll, werden 10 bis 20 Gew.-% Polysiloxan-polyalkyl-polyethercopolymere in Kombination mit organischen nichtionogenen Emulgatoren eingesetzt.

Im Vergleich zu den üblichen Rezepturen für die zu gießenden festen Puder-Zubereitungen weisen somit die Rezepturen für die spritzfähigen pastösen Massen weniger fettartige und wachsartige Bestandteile und weniger Nylon-Pulver auf, jedoch einen höheren Anteil Pigment bzw. Perlpigment bzw. als sonstigen Zusatz gemäß Merkmal e) Polysiloxan-polyalkyl-polyethercopolymere in Kombination mit organischen nichtionogenen Emulgatoren. Dadurch entstehen nicht fließbare, jedoch strangbildende pastöse Produkte, aus denen aus einer Spritzdüse Stege in die Behältnisse gespritzt werden können. Diese Stege können gerade, aber auch gebogen oder gekrümmt sein. Diese pastösen Stege schmelzen auch bei späteren Arbeitstemperaturen bis zu 80°C nicht wieder auf. Sie sind jedoch in der Lage, sich mit den flüssigen, gegossenen Puder-Zubereitungen nach dem Trocknungsvorgang zu einem einheitlichen, festen Puder zu verbinden. Sie bilden meist sogar eine optisch nicht oder kaum noch sichtbare Trennschicht, die sich bei der späteren Verwendung der Puder-Zubereitung in den Behältnissen völlig neutral verhalten. Da sie keine Farbpigmente, sondern nur farbneutrale Pigmente bzw. Perlpigmente enthalten, wird keine Vermischung der Farben beobachtet.

Nach dem erfindungsgemäßen Verfahren ist es somit möglich, in einem größeren Behältnis zwei oder mehrere voneinander getrennte verschiedenfarbige Teilbereiche herzustellen und dabei ansprechende Formen und Ornamente herzustellen. Das Verfahren ist dabei einfacher und preiswerter, als wenn Behältnisse mit Trennstegen hergestellt werden müßten, zumal für jede einzelne Formgebung neue Spritzwerkzeuge oder Tiefziehformen zur Verfügung gestellt werden müßten.

In den nachfolgenden Beispielen sind bevorzugte Rezepturen für die spritzfähige pastöse Masse angegeben, welche insbesondere für die bereits auf dem Markt befindlichen Handelsprodukte geeignet sind. Prinzipiell ist es aber möglich, gemäß EP-A-135 060 verschiedenartige feste, gegossene Puder-Zubereitungen herzustellen, so daß auch daran angepaßte andere spritzfähige pastöse Massen zusammengestellt und verwendet werden können.

Beispiel 1

Eine spritzfähige pastöse Masse zum Gießen von Stegen in Behältnisse hat folgende Zusammensetzung:

| | |
|---|---|
| Oleyl Erucate | 4,0 % |
| Stearinsäure-mono-ethanolamid | 1,0 % |
| Glycerylstearat /oder Oleate | 0,5 % |
| Nylon-12-Pulver | 8,0 % |
| farbneutrales Pigment/ | |
| Perlpigment | 43,15% |
| Octamethylcyclotetrasiloxan | 43,0 % |
| Methylhydroxytoluol | 0,05% |
| Methlychloroisothiazolinone | |
| + Methylisothiazolinone | 0,10% |
| Methylparaban | 0,10% |
| Propylparaban | 0,10% |
| | 100,00% |

Dieses Gemisch kann bei Raumtemperatur oder Temperaturen bis zu 40° aus einer Düse strangförmig in Behältnisse eingebracht werden, und zwar mit einem Strangdurchmesser der etwa der Schichtdicke des späteren Puders entspricht. Unmittelbar danach können sie in an sich bekannter Weise mit verschiedenfarbigen Puder-Zubereitungen ausgegossen werden. Die Behältnisse werden in einem Trockenschrank bei 60°C schichtweise eingelagert und hierin etwa 5 bis 10 Stunden belassen, wobei der größte Anteil des Octamethylcyclotetrasiloxans verdampft. Gewünschtenfalls wird die Oberfläche der so erhaltenen Produkte mit Drücken unter 10 bar bearbeitet.

Beispiel 2

Eine andere spritzfähige pastöse Masse zum Gießen von Stegen in Behältnisse hat folgende Zusammensetzung:

```
Bienenwachs                             7,0 %
Stearinsäure-mono-ethanolamid           7,0 %
mikrokristallines Hartwachs
(Lunacera(R) 80)                        7,0 %
Cetyl Dimethicone Copolyol,
Cetyl Dimethicone,
Polyglyceryl-3-Oleate,
Hexyl Laurate (Abil(R) WS 08)          15,0 %
Octamethylcyclotetrasiloxan            54,0 %
farbneutrales Pigment und/oder
Perlpigment                            10,0 %
                                      100,00%
```

Die Komponenten werden zunächst ohne das Lösungsmittel und das Pigment miteinander vermischt und aufgeschmolzen. Danach werden die Lösungsmittel und das Pigment dazugegeben und homogen vermischt. Dieses Gemisch wird, wie im Beispiel 1 beschrieben, bei Raumtemperatur oder Temperaturen bis 40°C aus einer Düse strangförmig in Behältnisse eingebracht und weiterverarbeitet. Dieses Gemisch läßt sich insbesondere bei höheren Drücken einwandfrei verarbeiten, ohne daß es zu Entmischungen kommt. Das Verhalten beim Trocknen entspricht dem der Pudermassen.

**Patentansprüche**

1. Verfahren zur Herstellung von Behältnissen mit zwei oder mehreren voneinander getrennten, verschiedenfarbigen Teilbereichen fester, gegossener Puder-Zubereitungen, dadurch gekennzeichnet, daß in die Behältnisse
    I. Trennstege eingespritzt werden aus einer pastösen Masse bestehend aus
        a) 0 bis 15 Gew.-% eines nicht hygroskopischen, rieselfähigen, organischen Feststoff mit einer Teilchengröße von 5 bis 50 μm Durchmesser als Füllstoff,
        b) einem oder mehreren fettartigen und wachsartigen Bestandteilen,
        c) einem farbneutralen Pigment oder Perlpigment,
        d) einem oder mehreren verdampfbaren untoxischen, hydrophoben Lösungsmitteln,
        e) gegebenenfalls weiteren üblichen Zusätzen für gießbare feste Puder-Zubereitungen,
        worauf
    II. in die einzelnen Teilbereiche der durch Stege unterteilten Behältnisse verschiedenfarbige Puder-Zubereitungen gegossen werden,
        worauf
    III. der Überschuß des Lösungsmittels verdampft und gewünschtenfalls die Oberfläche der so

4

EP 0 255 655 B1

erhaltenen Produkte mit Drücken unter 10 bar bearbeitet wird.

2. Verfahren gemäß Anspruch 1, dadurch gekennzeichnet, daß als a) Nylon-12-Pulver verwendet wird, bestehend aus überwiegend kugelförmigen und ellipsoiden Körpern.

3. Verfahren gemäß Anspruch 1 oder 2, dadurch gekennzeichnet, daß als b) Gemische verwendet werden aus Oleyl Erucate, Stearinsäure-mono-ethanolamid und Glyceryl Oleate oder Bienenwachs, Stearinsäure-mono-ethanolamid und mikrokristallinem Hartwachs.

4. Verfahren gemäß einem der Ansprüche 1 bis 3, dadurch gekennzeichnet, daß als d) verwendet wird Octamethylcyclotetrasiloxan.

5. Verfahren gemäß einem der Ansprüche 1 bis 4, dadurch gekennzeichnet, daß als e) verwendet wird Methylparaban, Propylparaban, Butylhydroxytoluol und Methylchloroisothiazolinone/Methylisothiazolinone oder Polysiloxan-polyalkyl-polyethercopolymere in Kombination mit organischen nichtionogenen Emulgatoren.

6. Verfahren gemäß einem der Ansprüche 1 bis 5, dadurch gekennzeichnet, daß als pastöse Masse verwendet wird ein Gemisch aus

| a) | 7,0 bis 15,0 Gew.-% | Nylon-12-Pulver, |
|---|---|---|
| b) | 3,0 bis 5,0 Gew.-%<br>0,8 bis 2,0 Gew.-%<br>0,4 bis 2,0 Gew.-% | Oleyl Erucate,<br>Stearinsäure-mono-ethanolamid,<br>Glycerylstearate und/oder Oleate, |
| c)<br>d) | 40 bis 50 Gew.-%<br>35 bis 45 Gew.-% | farbneutrales Pigment und/oder Perlpigment,<br>Octamethylcyclotetrasiloxan, |
| e) | ca. 0,1 Gew.-%<br>ca. 0,1 Gew.-%<br>ca. 0,1 Gew.-%<br>ca. 0,05 Gew.-% | Methylparaban,<br>Propylparaban,<br>Methylchloroisothiazolinone und Methylisothiazolinone,<br>Butylhydroxytoluol. |

7. Verfahren gemäß einem der Ansprüche 1 bis 5, dadurch gekennzeichnet, daß als pastöse Masse verwendet wird ein Gemisch aus:

| b) | 6 bis 8 Gew.-%<br>6 bis 8 Gew.-%<br>6 bis 8 Gew.-% | Bienenwachs<br>Stearinsäure-mono-ethanolamid<br>mikrokristallines Hartwachs |
|---|---|---|
| c)<br>d)<br>e) | 8 bis 15 Gew.-%<br>50 bis 60 Gew.-%<br>10 bis 20 Gew.-% | farbneutrales Pigment und/oder Perlpigment<br>Octamethylcyclotetrasiloxan<br>Polysiloxan-polyalkyl-polyethercopolymere in Kombination mit organischen nichtionogenen Emulgatoren |

## Claims

1. Process of fabrication of containers with two or more differently coloured, separated from each other, sections of solid, casted powder preparations characterized in that into the containers
   I. separating walls are injected of a pasty mass consisting of
      a) 0 to 15% by weight of a non-hygroscopic, flowable organic solid substance with a particle size of 5 to 50 $\mu$m diameter as filling agent,
      b) one or more fatlike and wax-like components,
      c) a neutral coloured pigment or pearl pigment,
      d) one or more vaporable, untoxic hydrophobic solvents,

5

e) if applicable, further usual additives for castable solid powder preparations, whereupon

II. into the single sections of the containers being subdivided by walls, different coloured powder preparations are poured, whereupon

III. the excess of the solvent is evaporated and the surface of the products recieved this way is treated with pressure below 10 bar, if desired.

**2.** Process according to claim 1 characterized in that as a) nylon-12-powder is used, consisting mainly of spheric and elliptic particles.

**3.** Process according to claim 1 or 2, characterized in that as b) mixtures are used of oleyl erucate, stearic acid-mono-ethanolamide and glyceryl oleate or beeswax, stearic acid-mono-ethanolamide and microcrystalline hard wax.

**4.** Process according to one of the claims 1 to 3, characterized in that as d) octamethyl cyclotetrasiloxane is used.

**5.** Process according to one of the claims 1 to 4, characterized in that as e) is used methylparabane, propylparabane, butylhydroxytoluene and methyl chloroisothiazolinone/methyl isothiazolinone or polysiloxane-polyalkyl-polyether copolymers in combination with organic non-ionic emulsifiers.

**6.** Process according to one of the claims 1 to 5, characterized in that as pasty mass is used a mixture of

| a) | 7,0 to 15,0% | by weight nylon-12-powder, |
|---|---|---|
| b) | 3,0 to 5,0%<br>0,8 to 2,0%<br>0,4 to 2,0% | by weight oleyl erucate,<br>by weight stearic acid mono-ethanolamide<br>by weight glycerylstearate and/or oleate |
| c)<br>d) | 40 to 50%<br>35 to 45% | by weight neutral coloured pigment or pearl pigment<br>by weight octamethylcyclotetrasiloxane |
| e) | about 0,1%<br>about 0,1%<br>about 0,1%<br>about 0,05% | by weight methylparabane<br>by weight propylparabane<br>by weight methyl chloroisothiasolinone et methyl isothiazolinone<br>by weight butylhydroxytoluene |

**7.** Process according to one of the claims 1 to 5, characterized in that as pasty mass a mixture is used of:

| b) | 6 to 8%<br>6 to 8%<br>6 to 8% | by weight beeswax<br>by weight stearic acid monoethanolamid<br>by weight microcrystalline hard wax |
|---|---|---|
| c) | 8 to 15% | by weight neutral coloured pigment and/or pearl pigment |
| d)<br>e) | 50 to 60%<br>10 to 20% | by weight octamethyl cyclotetrasiloxane<br>by weight polysiloxane-polyalkylpolyether copolymers in combination with organic non-ionic emulsifiers. |

## Revendications

**1.** Procédé de fabrication de récipients avec deux ou plusieurs domaines partiels, separés l'un de l'autre et de différentes couleurs, de solides préparations de poudre coulées, caractérisé en ce que dans les récipients

I. on injecte des passerelles d'une matière pâteuse consistant

6

a) de 0 à 15% en poids d'une matière organique, non hygroscopique, fluide avec une grosseur de particules de 5 à 50 $\mu$m diamètre comme charge,

b) d'un ou de plusieurs composés cirés et grasseux,

c) d'un pigment de couleur neutre ou pigment nacré,

d) d'un ou de plusieurs solvants évaporables, non-toxiques, hydrophobiques,

e) le cas échéant en plus d'additifs habituels pour de préparations de poudre solides et coulables, après quoi

II. on coule des préparations de poudre en couleurs différentes dans les domaines partiels isolés des récipients séparés par les passerelles, après qoui

III. l'excédent du solvant évapore et si on le désire, la surface des produits tellement reçus est soumise à des pressions au-dessous de 10 bar.

2. Procédé selon la revendication 1 caractérisé en ce que comme a) on utilise poudre de nylon-12, consistant de grains sphériques et ellipsoidaux.

3. Procédé selon la revendication 1 ou 2, caractérisé en ce que comme b) on utilise de mélanges d'érucate oléylique, de mono éthanolamide d'acide stéarique et d'oléate de glycéryle ou de cire d'abeille, de mono éthanolamide d'acide stéarique et de cire dure microcristalline.

4. Procédé selon une des revendications 1 à 3, caractérisé en ce que comme d) on utilise octaméthylcyclotetrasiloxane.

5. Procédé selon une des revendications 1 à 4, caractérisé en ce que comme e) on utilise méthylparabane, propylparabane, butylhydroxytoluène et methylchloroisothiazolinone/méthylisothiazolinone ou polyéthercopolymères de polysiloxane polyalkyl en combinaison d'émulsifiants organiques et non ionogènes.

6. Procédé selon une des revendications 1 à 5, caractérisé en ce que comme masse pâteuse on utilise un mélange de

| a) | 7,0 à 15,0% | en poids poudre de nylon 12 |
|----|-------------|------------------------------|
| b) | 3,0 à 5,0%<br>0,8 à 2,0%<br>0,4 à 2,0% | en poids érucate oléylique<br>en poids monoéthanolamide d'acide stéarique<br>en poids glycérylstéarates et/ou oléates |
| c)<br>d) | 40 à 50%<br>35 à 45% | en poids pigment de couleurs neutres ou pigment nacré<br>en poids octaméthylcyclotetrasiloxane |
| e) | environ 0,1%<br>environ 0,1%<br>environ 0,1%<br>environ 0,05% | en poids methylparabane<br>en poids propylparabane<br>en poids methylchloroisothiazolinone et méthylisothiazolinone<br>en poids buthylhydroxytoluène |

7. Procédé selon une des revendications 1 à 5, caractérisé en ce que comme masse pâteuse on utilise un mélange de:

| b) | 6 à 8%<br>6 à 8%<br>6 à 8% | en poids cire d'abeille<br>en poids mono éthanolamide d'acide stéarique<br>en poids cire dure microcristalline |
|----|-----------------------------|------------------------------------------------------------------------------------------|
| c)<br>d)<br>e) | 8 à 15%<br>50 à 60%<br>10 à 20% | en poids pigment de couleures neutres et/ou pigment nacré<br>en poids octaméthylcyclotetrasiloxane<br>en poids polyéthercopolymères de polysiloxane polyalkyl en combinaison d'émulsifiants organiques et non ionogènes. |